# EUROPEAN PATENT SPECIFICATION

(11) **EP 3 315 485 B1**
(45) Date of publication and mention of the grant of the patent: **02.09.2020**
(21) Application number: 15756459.2
(22) Date of filing: 30.06.2015
(51) Int. Cl.: C07C 29/86, C07C 31/22

(54) **CRUDE GLYCEROL PURIFICATION PROCESS**
VERFAHREN ZUR REINIGUNG VON ROHGYLYCEROL
PROCÉDÉ DE PURIFICATION DE GLYCÉROL BRUT

(30) Priority: 29.06.2015 PT 2015108590
(43) Date of publication of application: 02.05.2018
(73) Proprietor: Sapec Química, SA., 3881-901 Ovar (PT); Universidade de Aveiro, 3810-193 Aveiro (PT)
(72) Inventor: DE FARIA FERREIRA, Artur Jorge, P-3800-907 Aveiro (PT); MARQUES GODINHO, Bruno Miguel, P-3800-337 Aveiro (PT); BOTAS NEVES DOS SANTOS, Ricardo Jorge, P-3810-498 Aveiro (PT); DA COSTA GAMA BATISTA DE MELO, Nuno Vasco, P-3700-791 Ovar (PT); MADEIRA VIEGAS DE BARROS TIMMONS, Ana Margarida, P-3810-187 Aveiro (PT); MAGUETA DA SILVA, Rui Alberto, 3800-336 Aveiro (PT)
(74) Representative: Patentree
(86) International application number: PCT/IB2015/054907
(87) International publication number: WO 2017/001892

(56) References cited:
- CN-A- 102 503 014
- US-A- 2 194 665
- US-A- 2 436 209
- US-A- 4 560 812

## Description

### Technical domain

The present disclosure relates to a process for the desalting/purification of crude glycerol with high salt content, particularly with salt contents greater than 2% (m/m), preferably greater than 3% (m/m).

The present disclosure can also be used as an efficient crude glycerol precleaning process so as to obtain a crude glycerol with low salts concentration, which gives way to efficient use of ion exchange or membrane separation technologies for obtaining glycerol with very high purity degree.

### Background

In recent years, biodiesel-based fuel technology has been worldwide developed and applied as a viable alternative to fossil fuels. Crude glycerol is the main byproduct of biodiesel production. The increase in biodiesel production has resulted in a serious waste crisis, since 1 kg crude glycerol is obtained as byproduct per 10 kg of biodiesel produced (M. S. Ardi, M. K. Aroua, and N. A. Hashim, "Progress, prospect and challenges in glycerol purification process: A review," Renew. Sustain. Energy Rev., vol. 42, pp. 1164-1173, 2015.)

Biodiesel is produced by transesterification of vegetable oils and/or animal fat with either methanol or ethanol, catalyzed by a base such as sodium hydroxide or potassium hydroxide. In addition, fatty acids present with triglycerides must be neutralized by a basic catalyst. As a result, if the amount of fatty acids is high, at the end of the process, salt concentration and water content in crude glycerol shall be even greater. Typically, the resulting crude glycerol has various impurities in its composition, including fatty acid salts, alkoxide salts, inorganic salts, "Matter Organic Non-Glycerol" (MONG), water and unreacted alcohol. This glycerol with high level of impurities exceeding 10% m/m, has a reduced market value thus creating an inventory problem to biodiesel producers who have difficulties putting this byproduct in the market, accumulating it on their facilities. For this reason, finding solutions that add value to this byproduct is crucial. Moreover, high-purity glycerol has a significant commercial value for the market, particularly for food, pharmaceutical and cosmetic industries (M. S. Ardi, M. K. Aroua, and N. A. Hashim, "Progress, prospect and challenges in glycerol purification process: A review," Renew. Sustain. Energy Rev., vol. 42, pp. 1164-1173, 2015).

In order to address this market, crude glycerol purification processes require new, more efficient, faster and cheaper technologies.

The removal of salts from polyols has been since long studied. The application of liquid-liquid extraction techniques in order to concentrate, extract and recover polyols, such as glycerin from aqueous solutions has been patented in 1948 by E.J. Clifton from Colgate-Palmolive Company, having been revised in 1949 in US2436209. For glycerin aqueous solutions containing impurities such as inorganic salts, the document discloses that subsequently to glycerin aqueous solutions contacting with organic solvents, two phases are obtained, a glycerin-rich liquid phase and a salt-rich aqueous phase. The complete process involves successive liquid-liquid extractions with different organic solvents, but the purity degree of the glycerin obtained is not disclosed.

Recently in 2013, CN102503014 by Sufei et al. discloses the use of n-butanol for processing glycerol saline wastewater. Using n-butanol between 30 and 70 °C, and an extraction column, two phases are obtained: a top glycerol-rich phase and a bottom aqueous phase. This bottom phase is filtered, the filtrate being returned to the extraction column for reprocessing. After one of a number of several distillations of the upper phase under vacuum, glycerol is obtained with 99.9% purity.

However, despite these contributions, the purification of crude glycerol from biodiesel production is quite different from the purification of glycerin aqueous solutions. The purification technology of crude glycerol obtained as byproduct from biodiesel production consists of an adaptation of the technology used in soap industry, which uses distillations under reduced pressure, and other treatments such as ion exchange and passage through activated charcoal. Generally, the purification process typical from glycerol involves three steps: (i) removing extra glycerol substances, which can be accomplished during neutralizations with strong acids, wherein free fatty acids and certain salts are removed, (ii) evaporation of volatile elements (water, alcohols) and (iii) a final step consisting in glycerol refinement and which may include different combinations of purification methods. These include vacuum distillation, ion exchange, as well as membrane separation and adsorption. Techniques such as neutralization (use of strong acids), ion exchange and membrane technology have shown to be useful in removing of salts. In turn, the distillation under reduced pressure, allows for obtaining glycerol with a purity degree of 96.6% and a low ash content (0.03%). However, this process is very energy demanding. The amount of energy required for vaporization and thermal decomposition, makes the process expensive due to glycerol's high specific heat capacity.

As the composition of crude glycerol is variable and depends on the origin of the raw material used, the purification process is necessarily also different and must be adapted. Furthermore, selecting glycerol purification processes depends on the final application thereof, which is associated with purity degree and final costs. Where glycerol is primarily intended for technical applications, the presence of some contaminants is acceptable (> 96.6% glycerol). However, when use of glycerol is intended for food and pharmaceutical sectors, high purity is required (≥99,5% glycerol).

Regardless of the selected purification method, neutralization (use of strong acids) is generally used as a pre-treatment of crude glycerol so as to remove the catalyst and soaps. The reaction of the acid with the soaps results in free fatty acids and the reaction with the basic catalyst yields salts and water. This process is widely used as a first step and has proven to be quite useful. However, inorganic salts and free ions removal is incomplete, typically between 5 to 10% of the crude glycerol mass are salts and free ions.

Purification techniques based on ion exchange can remove various types of impurities from crude glycerol such as fatty acids, inorganic salts and free ions. The first commercial application of an ion exchange unit for purifying crude glycerol (soap industry) started in Los Angeles, California, in 1951, using three pairs of ion exchangers and one mixed bed. The glycerol thus obtained was of highest quality (99%) and comparable with glycerol obtained by distillation. However, it has been later discovered that the separation process was not effective due to the reduced number of ion exchange sites per unit volume. The use of acidic ion exchange resins has been adopted to adapt this technique to the purification of crude glycerol obtained from biodiesel production, in order to eliminate various types of salts such as fatty acid salts (5-50%), inorganic salts (1 -5%) and also free ions. The use of ion exchange resins is an efficient, low cost method and does not present difficulties in process scale-up. However, other aspects such as fouling, saturation and the necessary regeneration of resins need to be improved. Thus, the costs inherent to resin chemical regeneration become high for salt amounts between 5 to 7% and impractical for greater amounts, a range of values that occurs in crude glycerol.

An alternative technique for glycerol purification consists in the use of membranes. This process is based on the chemical potential differential and hydrostatic pressure between two phases separated by a membrane. These membranes act as a barrier separating and restricting the transport of various chemical elements in a selective manner. Several reports and some examples involving the series combination of different types of membranes have been published. Using this strategy with series of two or more nanofiltration or reverse osmosis membranes, a high-purity degree glycerol was obtained (99%) from crude glycerol from biodiesel production.

In order to take greater advantage of this technique, technologies involving the combination of different types of membranes have been developed. An example is the patented high efficiency electro-pressure Membrane (HEEPMTM), developed by EET Corporation. Also associated with crude glycerol purification, membrane distillation has been researched. The driving force of this separation technique is the vapor pressure gradient resulting from the temperature difference over the membrane. The main advantage of membrane distillation is associated with low fouling residues in the membranes. However, the major limitation is the low transmembrane flow, for which some solutions involving the combination with other membrane techniques have been proposed such as micro-, ultra- and nanofiltration, in order to overcome this problem. Despite all positive aspects of membrane technology, its use at an industrial level is not very widespread due to disadvantages such as fouling and the constant need for cleaning, the limited durability and the availability of membranes adapted to specific operations.

The use of electrodeionization technology - EDI - for the removal of salts from crude glycerol has been recently disclosed in WO 2010/033817 A2, by Haselow et al. EDI devices combine ion exchange resins, ion exchange membranes with direct current for ion removal. An EDI cell combines ion exchange and electrodialysis benefits, while minimizing the problems associated with the two separate technologies. The obtained purity of the glycerol may exceed 99%.

Document US 4560812 A discloses glycerine recovered from an aqueous waste stream containing but minor amounts of glycerine by the requested steps of; (1) evaporating, (2) contacting with pentanol, (3) separating precipitated salt, (4) flashing to remove pentanol, and (5) vacuum distillation.

Document US 2194665 A discloses methods of recovering polyhydric alcohols from aqueous solutions containing the same, specifically, methods of recovering glycerol from said solutions.

These facts are described to illustrate the technical problem solved by the embodiments of the present disclosure.

### General Description

One object of the present embodiments is to provide an alternative process for efficiently removing salts from crude glycerol in a few steps in order to become cost-effective.

Although several purification techniques are available, which allow obtaining high-purity glycerol from crude glycerol obtained as byproduct of biodiesel production, there remains a clear need towards developing a simple and efficient method to remove salts with unlimited concentration levels. The present disclosure describes a process by which to address this problem.

The present disclosure allows purifying crude glycerol by removing salts with high concentrations, preferably greater than 2% (m/m), more preferably greater than 3% (m/m), even more preferably greater than 5% (m/m); by extractive crystallization using tertiary alcohols selected from tert-butanol, tert-pentanol, or mixtures thereof. The crude glycerol used can be a byproduct from biodiesel production.

Secondary alcohols have the hydroxyl group bonded to a secondary carbon - i.e. a carbon atom which is bonded to only two other carbon atoms, such as 2-propanol (isopropanol).

Tertiary alcohols have a hydroxyl group bonded to a tertiary carbon, such as 2-methyl-2-propanol (tert-butanol), 2-methyl-2-butanol (tert-pentanol, trimethylcarbinol), or mixtures thereof.

It relates to an alternative process for the desalting/purification of crude glycerol with a salt content greater than 2% (m/m), preferably greater than 3% (m/m), more preferably greater than 5% (m/m) comprising the following steps:
mixing crude glycerol with a tertiary C3-C5 alcohol, wherein said tertiary alcohol is selected from a list consisting of tert-butanol, tert-pentanol, or
mixtures thereof;
allowing the formation of two phases of the above mixture;
separating the two phases and discarding the solid phase;
removing the alcohols from the liquid phase, particularly by evaporation, and
collecting the obtained glycerol.

In the process for the desalting/purification of crude glycerol, a tertiary C3-C5 alcohol, preferably a C4-C5 alcohol, wherein said tertiary alcohol is selected from a list consisting of tert-butanol, tert-pentanol, or mixtures thereof, is used.

In the process for the desalting/purification of crude glycerol, crude glycerol/alcohol mixture mass ratios are 1:5-1:3 inclusive even more preferably from 1:5-1:4.

In an embodiment of the process for the desalting/purification of crude glycerol, the separation of the two phases may be held by precipitation, filtration, centrifugation or combinations thereof.

In an embodiment of the process for the desalting/purification of crude glycerol, alcohols are recovered and/or recycled, in order to reduce solvent consumption and the environmental impact.

In an embodiment of the process for the desalting/purification of crude glycerol, the glycerol obtainable may also be subjected to further purification, in particular to vacuum distillation.

In an embodiment of the process for the desalting/purification of crude glycerol, it may previously be the crude glycerol to a pre-evaporation and/or pre-distillation.

In an embodiment, the process for the purification of crude glycerol with a salt content higher than 2% (m/m) comprises the following steps:
mixing crude glycerol with a tertiary C3-C5 alcohol;
wherein said tertiary alcohol is selected from a list consisting of: tert-butanol, tert-pentanol, or mixtures thereof;
wherein the crude glycerol/alcohol mass ratios are 1:5 -1:3, inclusive;
allowing the formation of two phases of the above mixture;
separating the two phases and discarding the solid phase;
removing the alcohols from the liquid phase, particularly by evaporation, and
collecting the obtained glycerol.

In an embodiment, in the process for the purification of crude glycerol with a salt content higher than 2% (m/m), the crude glycerol/alcohol mass ratios are 1:5.

### Detailed Description

The present disclosure relates to a novel process for crude glycerol purification, such as that resulting from biodiesel production, with high inorganic salts content, so as to obtain glycerol with low salts concentration. The process consists in precipitating the salts and other stained impurities by mixing the crude glycerol with a tertiary (tert) alcohol, selected from tert-butanol (2-methyl-2-propanol or t-butanol) or tert-pentanol, or mixtures thereof, in proportions ranging from 5 parts of alcohol to 1 part of crude glycerol (mass/mass), to 3:1, ideally 5:1. At the end of the mixture a liquid phase is obtained consisting of glycerol, alcohol, and soluble impurities in this solution, and a solid phase, consisting of inorganic salts and other stained impurities. This solid phase can easily be separated from the liquid phase by precipitation, filtration or centrifugation. The alcohol may be removed from the liquid phase by distillation to be reused, being the purified glycerol obtained as product, with a low salts content. This glycerol can be used as is or be subjected to further purification processes, such as ion exchange processes, to further increase the purity thereof.

This document describes the process of desalting crude glycerol by removing salts by extractive crystallization.

In an embodiment, the process consists of mixing crude glycerol with tertiary alcohols in order to promote salts precipitation. As a result, two phases are formed: (i) a liquid phase in the upper part, consisting of a glycerol mixture with tertiary alcohols; (ii) a solid phase at the bottom, consisting of salts and stained impurities.

Said alcohols, tested in tert-butanol, tert-pentanol extractive crystallization, have kosmotropic properties ("Kosmo-tropic" = order making). Kosmotropic agents are enhancing agents of hydrogen bonds. In other words, they are organizers of the water structure and of all substances that may create hydrogen bonds, such as glycerol. On the other hand, inorganic salts and free ions cannot create hydrogen bonds, and for this reason they precipitate in the presence of a kosmotropic agent.

Typically, crude glycerol has volatile organic compounds and water. In order to remove these compounds, such as alcohols and water, distillation/volatilization processes, either at low pressures or at ambient pressure, may be used. Temperatures between 90 and 105 °C are sufficient for this purpose without risk of degrading the glycerol. This step is optional but recommended to increase the efficiency of desalting. Due to this fact, in order to make desalting optimal, the number of extra components capable of creating hydrogen bonds should be minimal.

In an embodiment, the salts can be substantially removed from crude glycerol by mixing the crude glycerol with a tertiary alcohol, in particular tert-butanol (kosmotropic agent). Subsequently to the addition of tert-butanol, the solution becomes cloudy, and within a few seconds, there is a solid phase at the bottom and a liquid phase on top. The solid phase is rich in salts and stained impurities and the liquid phase consists of a desalted glycerol and tert-butanol mixture. The degree of desalting depends on the presence of additional elements with the ability to create hydrogen bonds (mainly water) and on the crude glycerol/tert-butanol proportion applied.

In an embodiment, the complete separation of solid and liquid phases may be accomplished by centrifugation, precipitation or by filtration. For example, at 1200 rpm perfect separation is achieved in less than 10 minutes. The liquid phase can have color, but it should be perfectly transparent after the separation is complete.

In an embodiment, the desalted glycerol and the alcohol, in particular tert-butanol can be separated by distillation. For example, a vacuum distillation (0.12159 bar) at 70 °C is sufficient to remove tert-butanol. The recovered tert-butanol can be reused in subsequent desaltings.

In an embodiment, tert-pentanol may also be used as an alternative to tert-butanol, but these substances are not recognized as being kosmotropic agents. In fact, for the same operating conditions, temperature and crude glycerol/alcohol ratio, the desalting efficiency with isopropanol is lower than that achieved using tert-butanol. However, with tert-pentanol desalting efficiency is slightly greater than that achieved with tert-butanol. As it can be seen in table 1. However, this superiority of desalting with tert-pentanol against tert-butanol is not observed, when only 3 parts of alcohol or less are applied. Furthermore, in a much hydrated crude glycerol, the desalting efficiency of tert-pentanol is lower than that of isopropanol for 3 or less parts of alcohol, since under these conditions tert-pentanol shows miscibility problems with crude glycerol.

Throughout the specification and claims the word "comprising" and variations thereof, are not intended to exclude other technical features, additives, components, or steps. Additional objects, advantages and features of the invention will become apparent to those skilled in the art upon examination of the specification, or may be learned upon practice of the invention. The following examples are provided by way of illustration and are not intended to be limiting of the present invention. Furthermore, the present invention covers all possible combinations of particular or preferred embodiments described herein.

### Exemplary embodiments

The present invention will be better understood after the following non-limiting examples for crude glycerol desalting.

### Example 1

### Crude glycerol 1:5 tert-butanol (m/m)

### Crude glycerol from biodiesel production

2.5 g crude glycerol (10.5% (m/m) water, 4.3% (m/m) salt) and 12.5 g tert-butanol were mixed for 1 min, in a vortex mixer, at room temperature. Some seconds after stirring was complete, the formation of two phases was observed: a liquid phase at the top (glycerol and tert-butanol mixture) and a solid phase, at the bottom (salts and stained impurities). For faster phase separation a centrifugation process has been used (1200 rpm, 10 min). A vacuum distillation (0.12159 bar, 70 °C, 30 min) of the top phase allowed separating tert-butanol from glycerol. Glycerol obtained by this procedure contained 0.5% water and 0.93% (m/m) salt. Thus, 0.089 g of salts were removed, corresponding to 82% desalting of crude glycerol.

### Example 2

### Pre-distilled crude glycerol 1:5 tert-butanol (m/m)

### Pre-distilled crude glycerol from biodiesel production

In this example, crude glycerol used was subjected to a pre-distillation step: 0.12159 bar pressure and a temperature of 90 °C for 30 min.

2.5 g crude glycerol (2.3 % (m/m) water, 5.0 % (m/m) salt) and 12.5 g tert-butanol were mixed for 1 min, in a vortex mixer, at room temperature. Some seconds after stirring was complete, the formation of two phases was observed: a liquid phase at the top (glycerol and tert-butanol mixture) and a solid phase, at the bottom (salts and stained impurities). For faster phase separation a centrifugation process has been used (1200 rpm, 10 min). A vacuum distillation (0.12159 bar, 70 °C, 30 min) of the top phase allowed separating tert-butanol from glycerol. Glycerol obtained by this procedure contained 0.5% (m/m) water and 0.82 % (m/m) salt. Thus, 0.107 g of salts were removed, corresponding to 85% desalting of crude glycerol.

### Example 3

### Pre-evaporated crude glycerol 1:5 tert-butanol (m/m)

### Pre-evaporated crude glycerol from biodiesel production

In this example, the crude glycerol used was subjected to a pre-evaporation step within a ventilated chamber (105 °C, 24 h).

2.5 g crude glycerol (0.5 % (m/m) water, 5.5 % (m/m) salt) and 12.5 g tert-butanol were mixed for 1 min, in a vortex mixer, at room temperature. Some seconds after stirring was complete, the formation of two phases was observed: a liquid phase at the top (glycerol and tert-butanol mixture) and a solid phase, at the bottom (salts and stained impurities). For faster phase separation a centrifugation process has been used (1200 rpm, 10 min). A vacuum distillation (0.12159 bar, 70 °C, 30 min) of the top phase allowed separating tert-butanol from glycerol. Glycerol obtained by this procedure contained 0.5% (m/m) water and 0.85 % (m/m) salt. Thus, 0.120 g of salts were removed, corresponding to 86% desalting of crude glycerol.

### Example 4

### Crude glycerol 1:5 tert-pentanol (m/m)

### Crude glycerol from biodiesel production

2.5 g crude glycerol (10.5 % (m/m) water, 4.3 % (m/m) salt) and 12.5 g tert-pentanol were mixed for 1 min, in a vortex mixer, at room temperature. Some seconds after stirring was complete, the formation of two phases was observed: a liquid phase at the top (glycerol and tert-pentanol mixture) and a solid phase, at the bottom (salts and stained impurities). For faster phase separation a centrifugation process has been used (1200 rpm, 10 min). A vacuum distillation (0.12159 bar, 70 °C, 30 min) of the top phase allowed separating tert-pentanol from glycerol. Glycerol obtained by this procedure contained 0.5% water and 0.66 % (m/m) salt. Thus, 0.093 g of salts were removed, corresponding to 87% desalting of crude glycerol.

### Example 5

### Pre-distilled crude glycerol 1:5 tert-pentanol (m/m)

### Pre-distilled crude glycerol from biodiesel production

In this example, crude glycerol used was subjected to a pre-distillation step: 0.12159 bar pressure and a temperature of 90 °C for 30 min.

2.5 g crude glycerol (2.3 % (m/m) water, 5 % (m/m) salt) and 12.5 g tert-pentanol were mixed for 1 min, in a vortex mixer, at room temperature. Some seconds after stirring was complete, the formation of two phases was observed: a liquid phase at the top (glycerol and tert-pentanol mixture) and a solid phase, at the bottom (salts and stained impurities). For faster phase separation a centrifugation process has been used (1200 rpm, 10 min). A vacuum distillation (0.12159 bar, 70 °C, 30 min) of the top phase allowed separating tert-pentanol from glycerol. Glycerol obtained by this procedure contained 0.5% (m/m) water and 0.72 % (m/m) salt. Thus, 0.110 g of salts were removed, corresponding to 87% desalting of crude glycerol.

### Example 6

### Pre-evaporated crude glycerol 1:5 tert-pentanol (m/m)

### Pre-evaporated crude glycerol from biodiesel production

In this example, the crude glycerol used was subjected to a pre-evaporation step within a ventilated chamber (105 °C, 24 h).

2.5 g crude glycerol (0.5 % (m/m) water, 5.3 % (m/m) salt) and 12.5 g tert-pentanol were mixed for 1 min, in a vortex mixer, at room temperature. Some seconds after stirring was complete, the formation of two phases was observed: a liquid phase at the top (glycerol and tert-pentanol mixture) and a solid phase, at the bottom (salts and stained impurities). For faster phase separation a centrifugation process has been used (1200 rpm, 10 min). A vacuum distillation (0.12159 bar, 70 °C, 30 min) of the top phase allowed separating tert-pentanol from glycerol. Glycerol obtained by this procedure contained 0.5% (m/m) water and 0.75 % (m/m) salt. Thus, 0.116 g of salts were removed, corresponding to 87% desalting of crude glycerol.

### Example 7 (comparative example)

### Crude glycerol 1:5 isopropanol (m/m)

### Crude glycerol from biodiesel production

2.5 g crude glycerol (10.5 % (m/m) water, 4.3 % (m/m) salt) and 12.5 g isopropanol were mixed for 1 min, in a vortex mixer, at room temperature. Some seconds after stirring was complete, the formation of two phases was observed: a liquid phase at the top (glycerol and isopropanol mixture) and a solid phase, at the bottom (salts and stained impurities). For faster phase separation a centrifugation process has been used (1200 rpm, 10 min). A vacuum distillation (0.12159 bar, 70 °C, 30 min) of the top phase allowed separating isopropanol from glycerol. Glycerol obtained by this procedure contained 0.5 water and 2.2 % (m/m) salt. Thus, 0.063 g of salts were removed, corresponding to 58% desalting of crude glycerol.

### Example 8 (comparative example)

### Pre-distilled crude glycerol 1:5 isopropanol (m/m)

### Pre-distilled crude glycerol from biodiesel production

In this example, crude glycerol used was subjected to a pre-distillation step: 0.12159 bar pressure and a temperature of 90 °C for 30 min.

2.5 g crude glycerol (2.3 % (m/m) water, 5 % (m/m) salt) and 12.5 g isopropanol were mixed for 1 min, in a vortex mixer, at room temperature. Some seconds after stirring was complete, the formation of two phases was observed: a liquid phase at the top (glycerol and isopropanol mixture) and a solid phase, at the bottom (salts and stained impurities). For faster phase separation a centrifugation process has been used (1200 rpm, 10 min). A vacuum distillation (0.12159 bar, 70 °C, 30 min) of the top phase allowed separating isopropanol from glycerol. Glycerol obtained by this procedure contained 0.5 water and 1.86 % (m/m) salt. Thus, 0.083 g of salts were removed, corresponding to 66% desalting of crude glycerol.

### Example 9 (comparative example)

### Pre-evaporated crude glycerol 1:5 isopropanol (m/m)

### Pre-evaporated crude glycerol from biodiesel production

In this example, the crude glycerol used was subjected to a pre-evaporation step within a ventilated chamber (105 °C, 24 h).

2.5 g crude glycerol (0.5 % (m/m) water, 5.5 % (m/m) salt) and 12.5 g isopropanol were mixed for 1 min, in a vortex mixer, at room temperature. Some seconds after stirring was complete, the formation of two phases was observed: a liquid phase at the top (glycerol and isopropanol mixture) and a solid phase, at the bottom (salts and stained impurities). For faster phase separation a centrifugation process has been used (1200 rpm, 10 min). A vacuum distillation (0.12159 bar, 70 °C, 30 min) of the top phase allowed separating isopropanol from glycerol. Glycerol obtained by this procedure contained 0.5 water and 1.94 % (m/m) salt. Thus, 0.095 g of salts were removed, corresponding to 68% desalting of crude glycerol.

**Table 1 - Analysis of salt removal efficiency with different alcohols and/or crude glycerol pretreatment (examples 7-9 are comparative examples)**

| | **Alcohol** | **Raw material Crude glycerol** | **Yield** |
|---|---|---|---|
| Example 1 | tert-butanol | Biodiesel production | 82% |
| Example 2 | tert-butanol | Pre-distilled | 85% |
| Example 3 | tert-butanol | Pre-evaporated | 86% |
| Example 4 | tert-pentanol | Biodiesel production | 87% |
| Example 5 | tert-pentanol | Pre-distilled | 87% |
| Example 6 | tert-pentanol | Pre-evaporated | 87% |
| Example 7 | isopropanol | Biodiesel production | 58% |
| Example 8 | isopropanol | Pre-distilled | 66% |
| Example 9 | isopropanol | Pre-evaporated | 68% |

The exemplary embodiments clearly show an increase in crude glycerol desalting/purification when tertiary alcohols are used. One may further observe the increased yield of the crude glycerol desalting/purification process when subjecting crude glycerol to previous treatments.

The embodiments described above are combinable.

The present disclosure is of course in no way restricted to the embodiments herein described and a person of ordinary skill in the art will be able to provide many modification possibilities thereto and substitutions of technical features for equivalents, according to requirements in each situation, as defined in the claims.

The following claims further define preferred embodiments.

## Claims

1. Process for the purification of crude glycerol with a salt content higher than 2% (m/m) comprising the following steps:
mixing crude glycerol with a tertiary C3-C5 alcohol;
wherein said tertiary alcohol is selected from a list consisting of: tert-butanol, tert-pentanol, or mixtures thereof;
wherein the crude glycerol/alcohol mass ratios are 1:5 -1:3, inclusive;
allowing the formation of two phases of the above mixture;
separating the two phases and discarding the solid phase;
removing the alcohols from the liquid phase, particularly by evaporation, and
collecting the obtained glycerol.

2. Process according to the previous claim wherein the crude glycerol/alcohol mass ratios are 1:5.

3. Process according to any of the previous claims wherein the separation of the two phases is by precipitation, by filtration, by centrifugation or combinations thereof.

4. Process according to any of the previous claims comprising recovering and/or recycling the removed alcohols.

5. Process according to any of the previous claims wherein the crude glycerol salt content is higher than 3% (m/m).

6. Process according to the previous claim wherein the crude glycerol salt content is higher than 5 % (m/m).

7. Process according to any of the previous claims comprising subjecting the obtained glycerol to a further purification, in particular by vacuum distillation.

8. Process according to any of the previous claims comprising previously subjecting the crude glycerol to a pre-evaporation and/or pre-distillation.

## Patentansprüche

1. Verfahren zur Reinigung von Rohglycerol mit einem Salzgehalt von mehr als 2 % (m/m), umfassend die folgenden Schritte:
Mischen von Rohglycerol mit einem tertiären C3-C5 Alkohol;
wobei der genannte tertiäre Alkohol ausgewählt wird aus einer Liste bestehend aus: tert-Butanol, tert-Pentanol oder Mischungen davon;
wobei die Rohglycerol/Alkohol-Massenverhältnisse 1:5 -1:3, einschließlich, betragen; die Bildung zweier Phasen der obigen Mischung erlauben;
Trennen der beiden Phasen und verwerfen der Festphase;
Entfernen der Alkohole aus der flüssigen Phase, insbesondere durch Verdampfen, und Sammeln des erhaltenen Glycerols.

2. Verfahren nach dem vorangehenden Anspruch, bei dem die Massenverhältnisse von Rohglycerol/Alkohol 1:5 betragen.

3. Verfahren nach einem der vorangehenden Ansprüche, bei dem die Trennung der beiden Phasen durch Ausfällung, durch Filtration, durch Zentrifugation oder Kombinationen davon erfolgt.

4. Verfahren nach einem der vorangehenden Ansprüche, dass die Rückgewinnung und/oder das Recycling der entfernten Alkohole umfasst.

5. Verfahren nach einem der vorangehenden Ansprüche, bei dem der Salzgehalt des Rohglycerols höher als 3 % (m/m) ist.

6. Verfahren nach dem vorangehenden Anspruch, bei dem der Salzgehalt des Rohglycerols höher als 5 % (m/m) ist.

7. Verfahren nach einem der vorangehenden Ansprüche, bei dem das erhaltene Glycerol einer weiteren Reinigung, insbesondere durch Vakuumdestillation, unterzogen wird.

8. Verfahren nach einem der vorangehenden Ansprüche, bei dem das Rohglycerol zuvor einer Voreindampfung und/oder Vordestillation unterzogen wird.

## Revendications

1. Procédé de purification de glycérol brut avec une teneur en sel supérieure à 2% (m/m) comprenant les étapes suivantes :
mélanger le glycérol brut avec un alcool tertiaire C3-C5 ;
dans lequel ledit alcool tertiaire est sélectionné à partir d'une liste consistant en :
tert-butanol, tert-pentanol, ou des mélanges de ceux-ci ;
dans lequel les ratios de masse de glycérol brut/alcool sont 1:5 - 1:3, inclut;
permettre la formation de deux phases du mélange ci-dessus ;
séparer les deux phases et éliminer la phase solide ;
retirer les alcools de la phase liquide, particulièrement par évaporation, et reccueillir le glycérol obtenu.

2. Procédé selon la revendication précédente dans lequel les ratios de masse de glycérol brut/alcool sont 1:5.

3. Procédé selon l'une quelconque des revendications précédentes dans lequel la séparation des deux phases est faite par précipitation, par filtration, par centrifugation ou des combinaisons de ceux-ci.

4. Procédé selon l'une quelconque des revendications précédentes comprenant la récupération et/ou le recyclage des alcools retirés.

5. Procédé selon l'une quelconque des revendications précédentes dans lequel la teneur en sel du glycérol brut est supérieure à 3% (m/m).

6. Procédé selon la revendication précédente dans lequel la teneur en sel du glycérol brut est supérieure à 5% (m/m).

7. Procédé selon l'une quelconque des revendications précédentes comprenant soumettre le glycérol obtenu à une autre purification, en particulier par distillation sous vide.

8. Procédé selon l'une quelconque des revendications précédentes comprenant soumettre préalablement le glycérol brut à une pré-évaporation et/ou pré-distillation.
